# EUROPEAN PATENT APPLICATION

(11) **EP 4 265 225 A1**
(43) Date of publication of application: **25.10.2023**
(21) Application number: 21908343.3
(22) Date of filing: 19.03.2021
(51) Int. Cl.: A61F 2/24

(54) **HEART VALVE PROSTHESIS AND STENT THEREOF, AND HEART VALVE PROSTHESIS REPLACEMENT SYSTEM**

(30) Priority: 21.12.2020 CN 202011521709; 21.12.2020 CN 202023104116 U
(71) Applicant: Hangzhou Valgen Medtech Co., Ltd., Hangzhou, Zhejiang 310051 (CN)
(72) Inventor: ZHANG, Tingchao, Hangzhou, Zhejiang 310052 (CN); XU, Li, Hangzhou, Zhejiang 310052 (CN); LI, Yang, Hangzhou, Zhejiang 310052 (CN)
(74) Representative: HWP Intellectual Property
(86) International application number: PCT/CN2021/081811
(87) International publication number: WO 2022/134330

(57) **Abstract**

A heart valve prosthesis stent (10), a heart valve prosthesis (100), and a heart valve prosthesis (100) replacement system. The heart valve prosthesis stent (10) comprises an inner stent (30) and an outer stent (40) are connected to each other. The outer stent (40) comprises an outer body section (41) and an outer skirt section (42) which extends from the outer body section (41) and is located on the radial outer side of the outer body section (41), wherein the outer skirt section (42) is located between an inflow end (410) and an outflow end (411) of the outer body section (41). The inner stent (30) comprises an inner body section (31) at least partially located in the outer stent (40), and an inner skirt section (32) protruding out of the radial outer side of the inner body section (31) from an inflow end (310) of the inner body section (31), wherein the inner skirt section (32) outwardly protrudes out of the radial outer side of the inflow end (410) of the outer body section (41), and the outer skirt section (42), the inner skirt section (32) and the section of the outer body section (41) located between the outer skirt section (42) and the inner skirt section (32) jointly form an accommodating space (50) with an outward opening in the radial direction. The heart valve prosthesis stent (10) can reduce damage to valvular annulus tissue (MVA).

## Description

### TECHNICAL FIELD

The present disclosure relates to the technical field of medical devices, in particular to a stent of prosthetic heart valve, a prosthetic heart valve with the stent, and a prosthetic heart valve replacement system.

### BACKGROUND

Heart valve plays an important role in the blood circulation of the heart, which acts as an one-way "valve" to prevent regurgitation. Both mitral valve, which prevents regurgitation of blood from the left ventricle (LV) to the left atrium (LA), and tricuspid valve, which prevents regurgitation of blood from the right ventricle (RV) to the right atrium (RA), are important heart valves and usually include valve annulus, leaflets, chordae tendineae and papillary muscles. During the ventricular systole, the leaflets of a normal mitral or tricuspid valve are tightly closed to fully block the blood flow from the ventricle to the atrium. However, the leaflets of a diseased mitral or tricuspid valve cannot be tightly closed, which results in mitral regurgitation (MR) or tricuspid regurgitation (TR).

In order to treat patients with valvular disease, especially patients with severe valvular disease, minimally invasive interventional procedure has always been used in recent years to implant a prosthetic heart valve into the native mitral or tricuspid of the heart to replace the diseased native valve. A prosthetic heart valve commonly includes a stent configured to be positioned at the native mitral or tricuspid valve of the heart, and prosthetic leaflets disposed within the stent which act as a one-way "valve" to prevent blood regurgitation. A prosthetic heart valve, which is positioned through barbs on the stent thereof which pierce into the valve annulus when implanted into the native mitral or tricuspid valve of the heart, is known in the art.

However, such a prosthetic heart valve, which is positioned by piercing the valve annulus with barbs, will cause injury to the valve annulus, and with the dynamic heartbeat, the injury is progressively increased.

### SUMMARY

### TECHNICAL PROBLEM

It is an object of the present disclosure to provide a stent of prosthetic heart valve, a prosthetic heart valve having the stent of prosthetic heart valve, and a prosthetic heart valve replacement system. The stent of prosthetic heart valve can reduce, or at least to some extent reduce, injury to the valve annulus.

### TECHNICAL SOLUTION

To this end, in one aspect, the present disclosure provides a stent of prosthetic heart valve, comprising an inner frame and an outer frame connected with each other; the outer frame comprises an outer body section, and an outer skirt section extending from the outer body section and located at a radially outer side of the outer body section, wherein the outer skirt section is located between an inflow end and an outflow end of the outer body section; the inner frame comprises an inner body section at least partially within the outer frame, and an inner skirt section projecting radially outside the inner body section from an inflow end of the inner body section, wherein the inner skirt section projects radially outside the inflow end of the outer body section, so that the outer skirt section, the inner skirt section, and a section of the outer body section between the outer skirt section and the inner skirt section together form a receiving space which opens radially outward.

In another aspect, the present disclosure provides a prosthetic heart valve, comprising at least two prosthetic leaflets and the aforementioned stent of prosthetic heart valve, wherein the prosthetic leaflets are fixedly connected within the inner body section of the inner frame of the stent of prosthetic heart valve, and edges of the at least two prosthetic leaflets are circumferentially engaged with each other.

In still another aspect, the present disclosure provide a prosthetic heart valve replacement system comprising the aforementioned prosthetic heart valve and a delivery device for deliver the prosthetic heart valve; the prosthetic heart valve has a delivery condition being radially compressed and a natural condition being radially expanded; the delivery device comprises an outer sheath and an inner core passing through the outer sheath, and the inner core and the outer sheath are axially movable relative to each other; the prosthetic heart valve is radially compressed and received in a gap between a distal portion of the inner core and a distal portion of the outer sheath.

### TECHNICAL ADAVANTAGE

The stent of prosthetic heart valve of the present disclosure has the receiving space which opens radially outward, which is formed by the outer skirt section located at the radially outer side of the outer body section, the inner skirt section projecting radially outside the inner body section, and the section of the outer body section between the outer skirt section and the inner skirt section. When the stent is implanted in a heart, the valve annulus can be received within the receiving space, with the section of the outer body section between the outer skirt section and the inner skirt section radially supporting the valve annulus, the outer skirt section is blocked by the valve annulus so as to prevent the stent of prosthetic heart valve from moving towards the atrial side, and the inner skirt section is blocked by the valve annulus to prevent the stent of prosthetic heart valve from moving towards the ventricular side, thereby positioning the stent of prosthetic heart valve without barbs piercing the valve annulus to achieve the positioning of the stent of prosthetic heart valve as in the prior art, which can at least reduce injury to the valve annulus to a certain extent.

### BRIEF DESCRIPTION OF THE DRAWINGS

In order to more clearly explain the embodiments of the present disclosure or the technical solutions in the prior art, a brief description for the drawings of the embodiments or the prior art is given below, and it will be apparent that, the drawings described below only show some embodiments of the disclosure, and other drawings can be obtained from these drawings without any creative labor for those of ordinary skill in the art, wherein
FIG. 1 is a perspective structural view of a prosthetic heart valve according to a first embodiment of the present disclosure;
FIG. 2 is a front view of the prosthetic heart valve shown in FIG. 1;
FIG. 3 is an exploded view of the prosthetic heart valve shown in FIG. 1;
FIG. 4a is a schematic view of the prosthetic heart valve of FIG. 1 implanted in a human heart with prosthetic leaflets and flow blocking films of the prosthetic heart valve not shown;
FIG. 4b is a partial enlarged view of FIG. 4a;
FIG. 4c is a schematic view of the prosthetic heart valve shown in FIG. 4a in engagement with the valve annulus;
FIG. 5 is a front view of the stent of prosthetic heart valve of the prosthetic heart valve shown in FIG. 1, with the flow blocking films not shown;
FIG. 6 is a front view of the inner frame of the stent of prosthetic heart valve shown in FIG. 5;
FIG. 7 is a front view of the outer frame of the stent of prosthetic heart valve shown in FIG. 5;
FIG. 8a is a top view of the outer body section of the outer frame shown in FIG. 7;
FIG. 8b shows another variant of the outer body section similar to that shown in FIG. 8a;
FIG. 9a is a front view of a position limiting rod of the outer frame shown in FIG. 7;
FIG. 9b shows another variant of the position limiting rod similar to that shown in FIG. 9a;
FIG. 9c shows a further variant of the position limiting rod similar to that shown in FIG. 9a;
FIG. 10a is a partial enlarged view of the outer frame shown in FIG. 7, showing a support unit consisting of two support rods;
FIG. 10b shows another variant of the support unit similar to that shown in FIG. 10a;
FIG. 10c shows a further variant of the support unit similar to that shown in FIG. 10a;
FIG. 10d shows a further variant of the support unit similar to that shown in FIG. 10a;
FIG. 11a shows another variant of the support unit similar to that shown in FIG. 10a, in which the support unit is substantially rod-shaped;
FIG. 11b shows another variant of the support unit similar to that in FIG. 11a;
FIG. 11c shows a further variant of the support unit similar to that in FIG. 11a;
FIG. 11d shows a further variant of the support unit similar to that in FIG. 11a;
FIG. 12 is a front view of the outer frame of the prosthetic heart valve according to a second embodiment of the present disclosure;
FIG. 13a is a partial enlarged view of FIG. 12;
FIG. 13b shows another variant of the marking mechanism similar to that shown in FIG. 13a;
FIG. 13c shows a further variant of the marking mechanism similar to that shown in FIG. 13a;
FIG. 14a is a front view of the outer frame of the prosthetic heart valve according to a third embodiment of the present disclosure;
FIG. 14b shows another variant of the outer frame similar to that shown in FIG. 14a;
FIG. 15 is a front view of the outer frame of the prosthetic heart valve according to a fourth embodiment of the present disclosure;
FIG. 16 is an exploded view of the outer frame shown in FIG. 15;
FIG. 17 is a front view of the prosthetic heart valve according to a fifth embodiment of the present disclosure;
FIG. 18 is an exploded view of the prosthetic heart valve shown in FIG. 17;
FIG. 19 is a schematic view of the prosthetic heart valve of FIG. 17 in engagement with the valve annulus;
FIG. 20 is a front view of the stent of prosthetic heart valve of the prosthetic heart valve according to a sixth of the present disclosure, with the flow blocking films not shown;
FIG. 21 is a front view of the outer frame of the stent of prosthetic heart valve shown in FIG. 20;
FIG. 22 is a schematic view of the stent of prosthetic heart valve shown in FIG. 20 in engagement with the valve annulus;
FIG. 23 is a front view of the stent of prosthetic heart valve of the prosthetic heart valve according to a seventh embodiment of the present disclosure, with the flow blocking films not shown;
FIG. 24 is a front view of the outer frame of the stent of prosthetic heart valve shown in FIG. 23;
FIG. 25 is a schematic view of the stent of prosthetic heart valve shown in FIG. 23 in engagement with the valve annulus; and
FIG. 26 is a schematic view of a prosthetic heart valve replacement system according to an embodiment of the present disclosure.

### DESCRIPTION OF THE EMBODIMENTS

In order to make the objects, technical solutions and advantages of the embodiments of the present disclosure more apparent, the technical solutions of the embodiments of the present disclosure will be described clearly and completely in combination with the drawings of the embodiments of the present disclosure. It should be noted that the embodiments described here are some but not all of the embodiments of the present disclosure. Based on the described embodiments of the disclosure, all other embodiments obtained by those skilled in the art without creative labor fall within the scope of protection of the present disclosure. In addition, the embodiments described below can be combined with each other as long as there is no contradiction or conflict between/among them, and the same or similar concepts or processes may not be described in detail in some embodiments.

First of all, it should be noted that the inflow end and the outflow end of the stent of prosthetic heart valve and the parts thereof, the prosthetic heart valve and the parts thereof are defined according to the direction of blood flow during the ventricular diastole, wherein the "inflow end" refers to the end close to the blood inflow side or close to the atrial side, and the "outflow end" refers to the end close to the blood outflow side or close to the ventricular side.

"Axial direction" refers to the direction parallel to an imaginary line connecting the center of the outflow end and the center of the inflow end. "Radial direction" refers to the direction perpendicular or substantially perpendicular to the axial direction. "Circumferential direction" refers to the direction around the axial direction. "Central axis" refers to an imaginary line connecting the center of the outflow end and the inflow end. "Upper", "above", "top" or the like refers to the orientation close to the inflow end. "Under", "below", "bottom" or the like refers to the orientation close to the outflow end. "Radial outside" refers to the side radially away from the central axis. "Radial inside" refers to the side radially close to the central axis.

"Inner" and "outer" are relative concepts referring to that one feature or the whole of an element on which the feature is located is at least partially radially inside or radially outside another feature or the whole of another element on which the another feature is located.

"Starting end" refers to a connection end of a feature for connecting with an adjacent feature. The "terminal end" refers to an end of a feature opposite to its starting end, and in some cases the terminal end is a "free end".

"Proximal end" refers to an end of a device or element close to the operator. "Distal end" refers to an end of the device or element away from the operator.

It should be noted that the above terms for indicating orientation or positional relationship are only for the purpose of making the description of the present disclosure clear and simple, rather than indicating or implying that the device or element referred to must have a special orientation, or be constructed and operated in a special direction, and thus are not to be construed as limiting the disclosure.

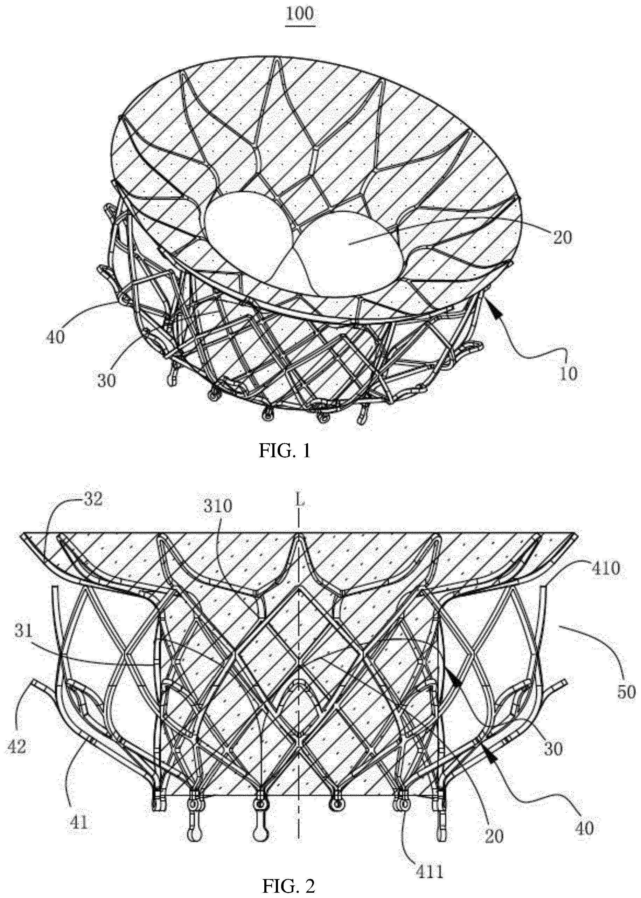

Referring to FIGS. 1 to 3, a prosthetic heart valve 100 according to an embodiment of the present disclosure includes a stent of prosthetic heart valve 10, and at least two prosthetic leaflets 20 disposed in the stent of prosthetic heart valve 10. The stent of prosthetic heart valve 10 includes an inner frame 30 and an outer frame 40 that are connected to each other. Preferably, the inner frame 30 is disposed coaxially within the outer frame 40. In other words, the central axis of the inner frame 30 described herein coincides with the central axis of the outer frame 40, both designated herein as L.

The stent of prosthetic heart valve 10 of the present disclosure has a delivery condition in which the stent is radially compressed and a natural condition in which the stent is radially expanded. In the delivery condition, the stent of prosthetic heart valve 10 is radially compressed by an external force, so that it can be compressed into a sheath with a small radial dimension, and thus delivered to the heart by a delivery device. In the natural condition, the stent of prosthetic heart valve 10 is released from the external force and is radially self-expanded. The following description refers to the structural features of the stent of prosthetic heart valve 10 in the natural condition, unless otherwise specified.

The outer frame 40 includes an outer body section 41, and an outer skirt section 42 extending from the outer body section 41 and located at a radially outer side of the outer body section 41. The outer skirt section 42 is located between an inflow end 410 and an outflow end 411 of the outer body section 41.

The inner frame 30 includes an inner body section 31 that is disposed at least partially within the outer frame 40, and an inner skirt section 32 projecting radially outside the inner body section 31 from an inflow end 310 of the inner body section 31. Furthermore, the inner skirt section 32 projects radially outside the inflow end 410 of the outer body section 41, so that the inner skirt section 32, the outer skirt section 42, and the section of the outer body section 41 between the outer skirt section 42 and the inner skirt section 32 together forms a receiving space 50 which is opened radially and outwardly.

It should be noted that, as mentioned above, "inner" and "outer" are relative concepts referring to that one feature or the whole of an element on which the feature is located is at least partially radially inside or radially outside another feature or the whole of another element on which the another feature is located, so that the aforementioned inner skirt section 32 is not meant to be located radially inside the outer skirt section 42, but the whole of the inner frame 30 on which the inner skirt section 32 is located is at least partially radially inside the whole of the outer frame 40 on which the outer skirt section 42 is located.

In this embodiment, the at least two prosthetic leaflets 20 are disposed within the inner body section 31 and are fixedly connected with the inner body section 31, for example, by sewing, and the edges of the at least two prosthetic leaflets 20 are circumferentially engaged with each other. The prosthetic leaflets 20 are preferably made of biological materials such as bovine pericardium and porcine pericardium. Alternatively, the prosthetic leaflets 20 can also be made of polymeric materials such as ultra-high molecular weight polyethylene.

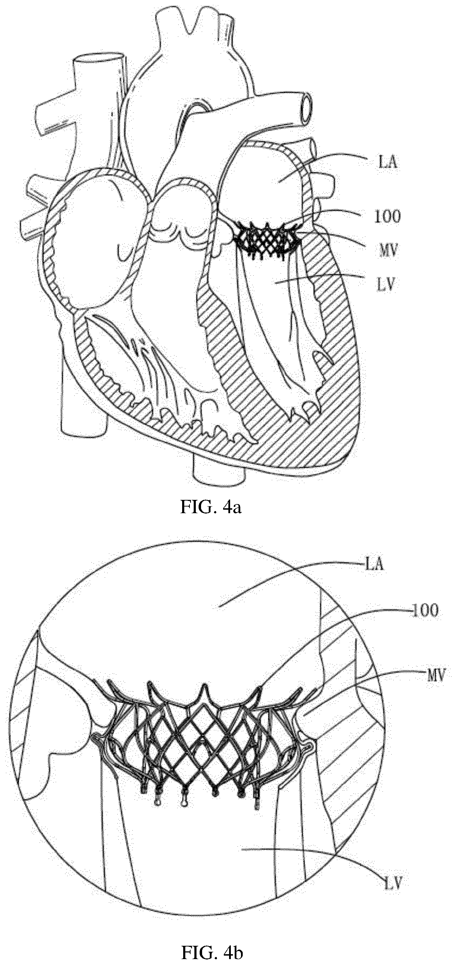

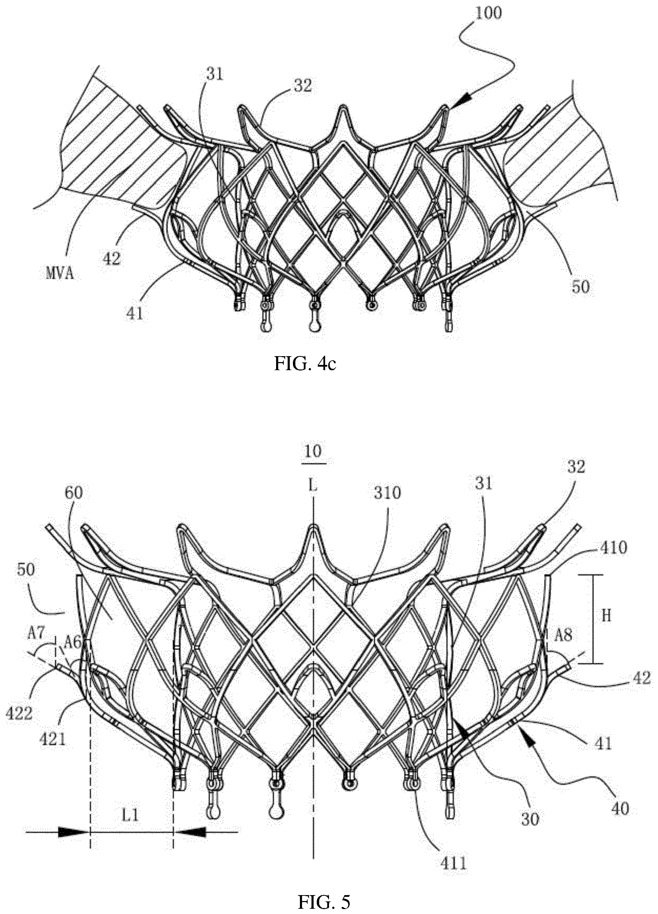

Referring to FIGS. 4a to 4c, the prosthetic heart valve 100 is implanted into a native valve of the heart to be replaced, for example at the mitral valve MV between the left atrium LA and the left ventricle LV. And the corresponding valve annulus MVA is received in the receiving space 50 of the stent of prosthetic heart valve 10, thereby positioning the prosthetic heart valve 100. During the systole of the left ventricular LV, the at least two prosthetic leaflets 20 are closed tightly to prevent regurgitation of blood from the left ventricle LV to the left atrium LA, and during the diastole of the left ventricular LV, the at least two prosthetic leaflets 20 are opened to allow blood to flow from the left atrium LA to the left ventricle LV.

It can be seen that the prosthetic heart valve 100 according to this embodiment receives the valve annulus MVA in the receiving space 50 of the stent 10, in which the section of the outer body section 41 of the stent of prosthetic heart valve 10 between the outer skirt section 42 and the inner skirt section 32 radially supports the valve annulus MVA, the outer skirt section 42 is blocked by the valve annulus MVA to prevent the stent of prosthetic heart valve 10 from moving towards the atrium, and the inner skirt section 32 is blocked by the valve annulus MVA to prevent the stent of prosthetic heart valve 10 from moving towards the ventricle, so as to position the prosthetic heart valve 100. There is no need to pierce the valve annulus MVA with any barbs as in the prior art to position the stent of prosthetic heart valve, thereby reducing injury to the valve annulus MVA.

It can be understood that the prosthetic heart valve 100 can be implanted into the heart through minimally invasive interventional procedure. For example, a delivery device which will be described in detail below can be used to receive the radially compressed prosthetic heart valve 100, and delivery the prosthetic heart valve 100 to the mitral valve via the apex of the heart, via the atrium, or via the femoral vein - the superior vena cava - the right atrium - the atrial septum - the left atrium, and then release the prosthetic heart valve 100 to replace the diseased native valve.

Alternatively, the prosthetic heart valve 100 can be implanted directly into the heart by other means such as surgical operation to replace the diseased native valve.

It will also be appreciated that the prosthetic heart valve 100 as shown in FIGS. 4a to 4c is only exemplarily shown for replacing the diseased native mitral valve, and in other embodiments, the prosthetic heart valve 100 can be used to replace other appropriate native valves, such as the tricuspid valve.

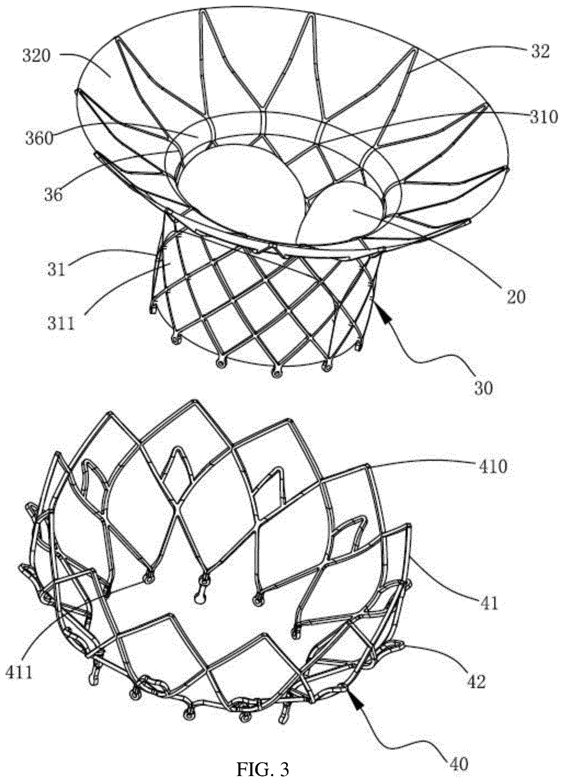

Referring to FIGS. 3, 5 and 6, in this embodiment, the inner body section 31 of the inner frame 30 is substantially in the shape of a hollow cylinder with two opened ends. Preferably, the outer diameter of the inner body section 31 ranges from 25 mm to 30 mm. It is further preferred that the inner body section 31 is covered with a flow blocking film 311. Preferably, the material of the flow blocking film 311 is PET, PTFE, or the like.

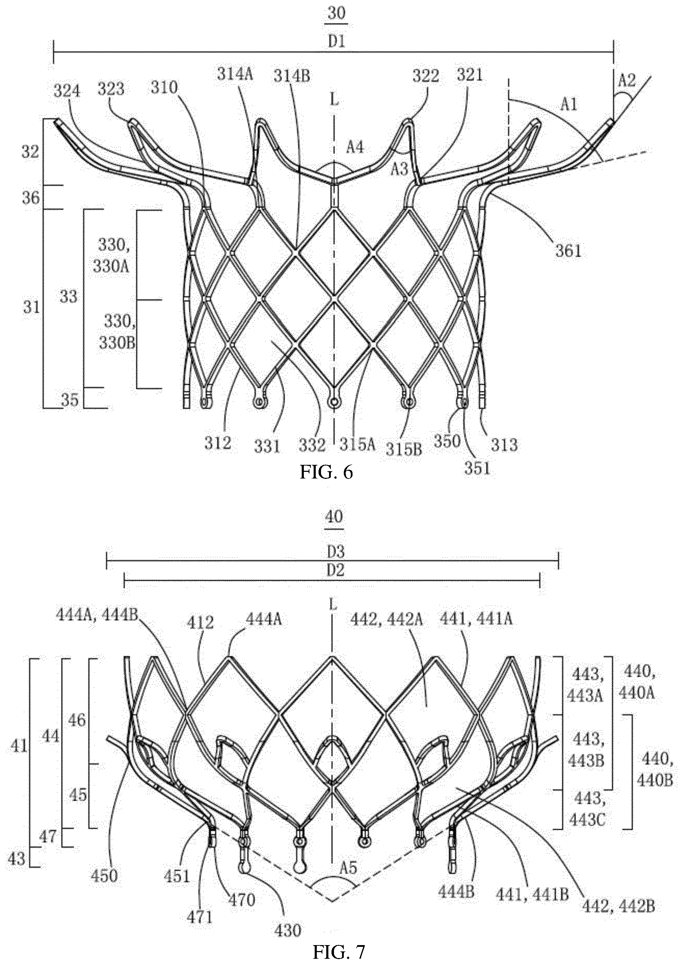

In this embodiment, the inner body section 31 includes an inner mesh structure 33 formed by a plurality of struts 312 which are connected with each other in a cross manner. The inner mesh structure 33 includes a plurality of inner annular units 330 (for example, FIG. 6 shows two inner annular units 330A and 330B) arranged in the axial direction. Each inner annular unit 330 includes a plurality of cells 331 arranged in the circumferential direction. Each cell 331 is enclosed by a plurality of struts 312 and has an opening 332. Preferably, each inner annular unit 330 is formed by a plurality of rhombic cells 331 arranged in the circumferential direction, wherein the struts 312 of the upper inner annular unit 330A close to the inflow end 310 of the inner body section 31 form a plurality of peaks 314A and valleys 314B that are alternatively distributed in the circumferential direction, and the struts 312 of the lower inner annular unit 330B close to the outflow end 313 of the inner body section 31 form a plurality of peaks 315A, valleys 315B that are alternatively distributed in the circumferential direction.

In this embodiment, the inner body section 31 further includes an inner connection structure 35 provided at the outflow end of the inner mesh structure 33 for connecting with the outer frame 40 (the corresponding connection structure of the outer frame 40 will be described below). In this embodiment, the inner connection structure 35 includes a plurality of inner connection units 350 that are spaced from each other in the circumferential direction. Each of the valleys 315B at the outflow end of the inner mesh structure 33 is connected with an inner connection unit 350. In this embodiment, each of the inner connection units 350 is substantially rod-shaped, and extends axially downward from a corresponding valley 315B at the outflow end of the inner mesh structure 33 and is enlarged at its terminal end with an inner connection hole 351 radially passing therethrough.

In this embodiment, the inner skirt section 32 of the inner frame 30 has a flared shape. The inner skirt section 32 is covered with a flow blocking film 320 for sealing at the atrial side and preventing perivalvular leakage. The material of the flow blocking film 320 is preferably PET, PTFE, or the like. Preferably, the inner skirt section 32 extends from the inflow end 310 of the inner body section 31 outwardly away from the central axis L of the inner frame 30 and axially away from the inflow end 310 of the inner body section 31. As shown in FIG. 6, the inner skirt section 32 extends obliquely upwards and outwards.

Preferably, the angle A1 formed between the starting end 321 of the inner skirt section 32 (i.e., the end close to the inner body section 31, and also the connection end) or the tangent line to the starting end 321 of the inner skirt section 32 and the central axis L of the inner frame 30 is in the range of 45 degrees to 90 degrees, so that the inner skirt section 32 can adapt to the valve annulus MVA better, thereby reducing the risk of perivalvular leakage. Preferably, the oblique angle A2 of the terminal end 322 of the inner skirt section 32 (i.e., the end away from the inner body section 31, and also the free end) with respect to the central axis L of the inner frame 30 is smaller than the oblique angle Al of the starting end 321 of the inner skirt section 32 with respect to the central axis L of the inner frame 30, so that the inner skirt section 32 can conform to the valve annulus MVA better, thereby further improving the positioning stability of the stent of prosthetic heart valve 10 and reducing the risk of perivalvular leakage.

Preferably, the diameter D1 of the terminal end 322 of the inner skirt section 32 ranges from 40 mm to 70 mm, which is about 10 mm greater than the inner diameter of a normal native valve annulus MVA, so that the prosthetic heart valve 100 can be effectively prevented from being displaced to the ventricular side and the risk of perivalvular leakage can be reduced.

Optionally, the inner skirt section 32 of the inner frame 30 includes a plurality of inner skirt units 323 which are distributed in the circumferential direction. Each inner skirt unit 323 includes two struts 324. The starting ends 321 of the two struts 324 are directly or indirectly connected with respective peaks 314A at the inflow end of the inner mesh structure 33, and the terminal ends 322 of the two struts 324 are connected with each other. The included angle A3 between the two struts 324 of each inner skirt unit 323 preferably ranges from 30 degrees to 150 degrees. Preferably, the two struts 324 of each inner skirt unit 323 are directly or indirectly connected with two adjacent peaks 314A at the inflow end of the inner mesh structure 33. More preferably, two adjacent struts 324 of two adjacent inner skirt units 323 of the inner skirt section 32 are joined together, i.e., corresponding to the same peak 314A at the inflow end of the inner mesh structure 33. The included angle A4 between two adjacent struts 324 of two adjacent inner skirt units 323 preferably ranges from 30 degrees to 150 degrees. It can be seen that all of the peaks 314A at the inflow end of the inner mesh structure 33 are directly or indirectly connected with respective struts 324 of the inner skirt section 32. It will be appreciated that, in other embodiments, the inner skirt section 32 can be configured differently as long as it can be supported at the atrial side of the valve annulus MVA.

Preferably, the inner frame 30 further includes a connection section 36 for connecting the inner skirt section 32 and the inner body section 31. Preferably, the connection section 36 is covered with a flow blocking film 360. The material of the flow blocking film 360 is preferably PET, PTFE, or the like. In this embodiment, the connection section 36 includes a plurality of connection rods 361 that are spaced from each other in the circumferential direction. One end of the connection rod 361 is connected with the joint of two adjacent inner skirt units 323 of the inner skirt section 32, and the other end is connected with a corresponding peak 314A at the inflow end of the inner mesh structure 33 of the inner body section 31. Preferably, the connection rod 361 transitions from the corresponding peak 314A at the inflow end of the inner mesh structure 33 to the joint of two adjacent inner skirt units 323 in an arc form, so as to prevent the connection rod 361 from being broken off.

Optionally, the inner skirt section 32, the inner body section 31, and the connection section 36 can be formed separately and respectively, and the inner skirt section 32 and the inner body section 31 are connected through the connection section 36, for example by crimping, riveting, welding or sewing.

Preferably, the inner skirt section 32, the inner body section 31, and the connection section 36 are formed in a single piece. That is, the inner frame 30 is preferably formed in an one piece.

Optionally, the inner frame 30 or the sections of the inner frame 30 can be formed by laser cutting a tube(s) having shape memory property such as a nickel-titanium tube (s) and heat setting the same into the desired shape. Alternatively, the inner frame 30 or the sections of the inner frame 30 can be formed by braiding a wire(s) having shape memory property such as a nickel-titanium alloy wire(s) and heat setting the same into the desired shape.

Referring to FIGS. 3, 5 and 7, in this embodiment, the inflow end 410 of the outer body section 41 of the outer frame 40 is free and suspended, and a radial gap 60 is formed between the section of the outer body section 41 between the outer skirt section 42 and the inner skirt section 32, which section radially supports the valve annulus and can be referred to as a support section, and the inner body section 31. The radial distance L1 of the radial gap 60 preferably ranges from 1 mm to 18 mm, more preferably from 5 mm to 18 mm. The radial gap 60 allows the support section of the outer frame 40 to be radially spaced from the inner body section 31 of the inner frame 30. When the outer frame 40 of the prosthetic heart valve 100 that has been implanted in the heart is deformed inward by the radial pressure from the valve annulus MVA, the radial gap 60 provides sufficient space for the deformation of the support section of the outer frame 20, in such a way that the deformed outer frame 40 under the radial pressure of the valve annulus would not interfere the inner skirt section 32 of the inner frame 30, thereby avoiding a gap between the inner skirt section 32 and the atrial side of the valve annulus due to deformation of the outer frame 40. Furthermore, the flow blocking film 320 covering the inner skirt section 32 can effectively isolate the atria from the ventricle, ensure the sealing at the atria side, and reduce the risk of perivalvular leakage.

In this embodiment, the outer body section 41 of the outer frame 40 includes an outer mesh structure 44 formed by a plurality of struts 412 which are connected with each other in a cross manner. The outer mesh structure 44 includes a bottom section 45 extending outwardly away from the central axis L of the outer frame 40 and towards the inner skirt section 32, and a top section 46 extending from the inflow end of the bottom section 45 further towards the inner skirt section 32.

In this embodiment, the bottom section 45 is substantially funnel-shaped, which gradually converges toward the central axis L of the outer frame 40 from the inflow end 450 toward the outflow end 451 thereof. The greater the convergence angle is, i.e., the greater the inclination of the bottom section 45 with respect to the central axis L of the outer frame 40 is, the shorter the axial length of the bottom section 45 is. Vice versa, the smaller the convergence angle is, i.e., the smaller the inclination of the bottom section 45 with respect to the central axis L of the outer frame 40 is, the longer the axial length of the bottom section 45 is. In order to reduce the overall height of the outer frame 40, it is preferred that the angle A5 formed between two tangent lines of the outflow end 451 of the bottom section 45 that are radially opposite to each other ranges from 90 degrees to 150 degrees.

Referring to FIG. 3, FIG. 5, FIG. 7 and FIG. 8a, in this embodiment, the top section 46 is substantially in the shape of a hollow cylinder with two opened ends, and the outer peripheral profile of the top section 46 is O-shaped, which helps to simplify the manufacturing process of the outer frame 40. In other words, any portions of the top section 46 in this embodiment substantially have the same diameter. Preferably, the diameter D2 of the top section 46 ranges from 30 mm to 60 mm, which is approximately equal to the inner diameter of the native valve annulus MVA, helping the valve annulus MVA to stably abut against the top section 46 radially.

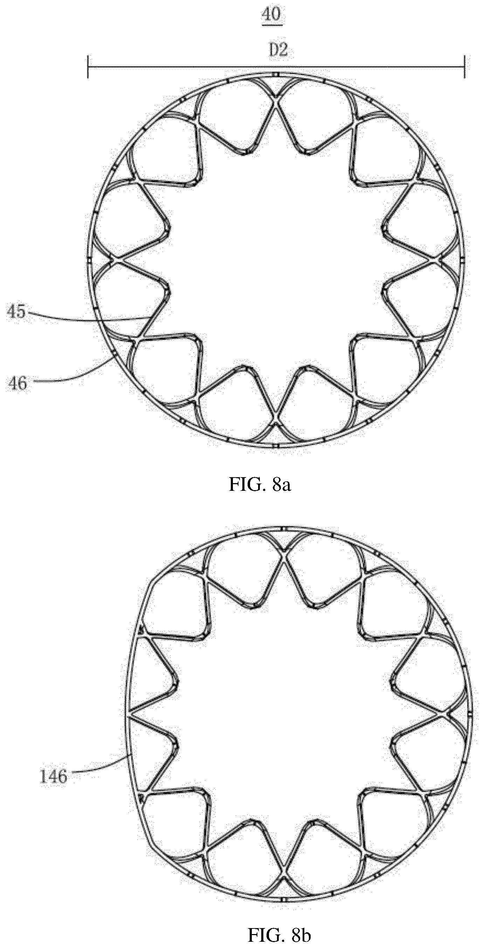

Referring to FIG. 8b, in other embodiments, the outer peripheral profile of the top section 146 can have other shapes, such as a "D" shape, which helps the top section 146 better conform to the native valve annulus MVA, avoiding pressing the ventricular outflow tract.

Referring again to FIGS. 3, 5 and 7, in this embodiment, the outer mesh structure 44 of the outer body section 41 is formed by a plurality of outer annular units 440 arranged in the axial direction. Each outer annular unit 440 is formed by a plurality of cells 441 arranged in the circumferential direction, wherein each cell 441 is enclosed by a plurality of struts 412 and has an opening 442.

Preferably, the outer mesh structure 44 of the outer body section 41 has a lower deformation resistance than the inner mesh structure 33 of the inner body section 31, so that the outer frame 40 can better conform to the native valve annulus MVA, while the inner frame 30 is able to subject to the dragging force from the prosthetic leaflets 20 without being easily deformed. The aforementioned deformation resistance refers to the ability to resist the deformation from the external stress, and under the same stress, the higher ability to resist the deformation, the less the deformation, and vice versa. In order to allow the inner body section 31 to have a higher deformation resistance than the outer body section 41, for example, the area of the openings 442 of the cells 441 of the outer mesh structure 44 can be larger than the area of the openings 332 of the cells 331 of the inner mesh structure 33. It can be understood that, in other embodiments, other techniques can be taken to allow the inner body section 31 to have a higher deformation resistance than the outer body section 41. For example, the inner body section 31 and the outer body section 41 can be made of materials with different rigidities, or consist of struts with different sizes, etc. It can be understood that the higher the rigidity of the material, the higher the deformation resistance, and the larger the strut size, the higher the deformation resistance.

In this embodiment, the outer mesh structure 44 consists of two outer annular units 440 which are offset from each other so that one cell 441 of each outer annular unit 440 is adjacent to two adjacent cells 441 of the other adjacent outer annular unit 440. Preferably, the area of the openings 442A of the cells 441A of the upper outer annular unit 440A which is mainly used to form the top section 46 is larger than the area of the openings 442B of the cells 441B of the lower outer annular unit 440B which is mainly used to form the bottom section 45, such that the deformation resistance of the top section 46 is lower than the deformation resistance of the bottom section 45, which increases the flexibility of the top section 46, thereby improving the compliance of the top section 46 to the valve annulus MVA, and also enhances the deformation resistance of the bottom section 45 to maintain its shape without being easily deformed. It can be understood that, in other embodiments, other techniques can be taken to allow the top section 46 to have a lower deformation resistance than the bottom section 45. For example, the top section 46 and the bottom section 45 can be made of materials with different rigidities, or consist of struts with different sizes, etc. It can be understood that the higher the rigidity of the material, the higher the deformation resistance, and the larger the strut size, the higher the deformation resistance.

In this embodiment, each outer annular unit 440 is formed by a plurality of rhombic cells 441 arranged in the circumferential direction. In other words, each outer annular unit 440 is formed by two layers of wave bars 443 that are connected in the axial direction, and each layer of the wave bar 443 is formed by a plurality of struts 412 that are circumferentially connected end to end and has a plurality of peaks 444A and valleys 444B that are alternately distributed in the circumferential direction. The two outer annular units 440A, 440B share the same middle wave bar 443B.

Preferably, the width of the struts 412 (i.e., the distance between the two long sides of the radially inner side or the radially outer side of each strut 412) of the three layers of wave bars 443 of the outer mesh structure 44 increases from the inflow end of the outer mesh structure 44 to the outflow end of the outer mesh structure 44, which further improves the compliance of the top section 46, thereby further enhancing the compliance of the top section 46 to the valve annulus MVA. More preferably, the width of any strut 412 of the outer mesh structure 44 is no more than 0.5 mm. Most preferably, the width of the struts 412 of the upper wave bar 443A, the middle wave bar 443B, and the lower wave bar 443C of the outer mesh structure 44 are 0.3 mm, 0.4 mm, and 0.5 mm, respectively.

In this embodiment, the outer body section 41 of the outer frame 40 further includes an outer connection structure 47 provided at the outflow end of the outer mesh structure 44 for connecting with the inner connection structure 35 of the inner frame 30. The outer connection structure 47 includes a plurality of outer connection units 470 that are spaced from each other in the circumferential direction. Preferably, each valley 444B at the outflow end of the outer mesh structure 44 is connected with an outer connection unit 470. In this embodiment, each of the outer connection units 470 is substantially rod-shaped and extends axially downward from a corresponding valley 444B at the outflow end of the outer mesh structure 44 and is enlarged at its terminal end with an outer connection hole 471 radially passing therethrough.

When connecting the outer frame 40 and the inner frame 30, the operator can place the inner frame 30 inside the outer frame 40 such that the outer connection holes 471 are aligned with the corresponding inner connection holes 351, and then insert connection members such as connection pins into the corresponding outer connection holes 471 and the corresponding inner connection holes 351, thereby connecting the outer frame 40 and the inner frame 30. It will be appreciated that, in other embodiments, the outer frame 40 and the inner frame 30 can be connected by using other techniques such as by sewing.

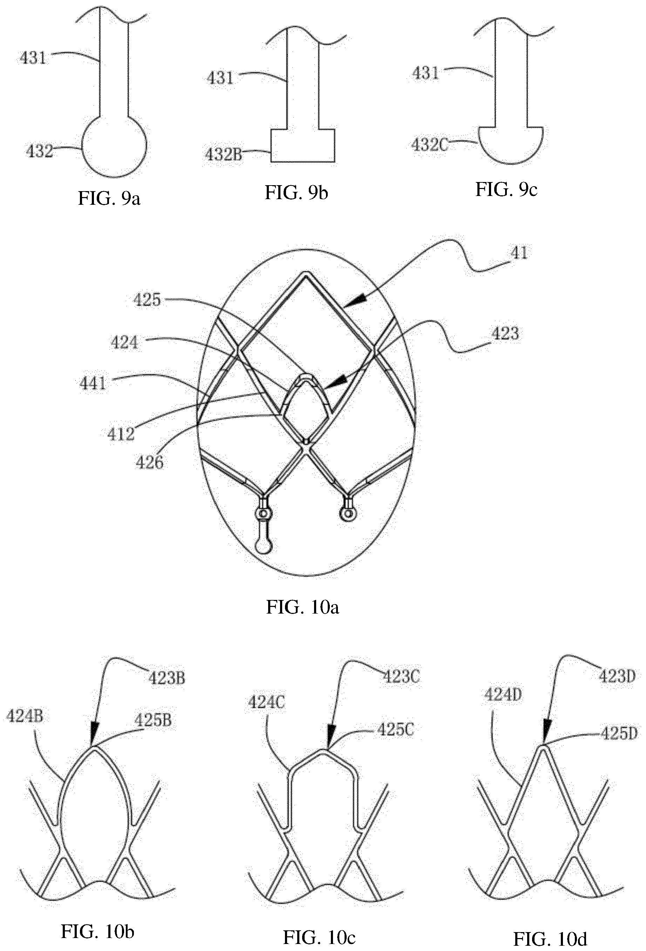

In this embodiment, the outer frame 40 further includes a position limiting structure 43 provided at the end of the outer connection structure 47 for connecting with the delivery device which will be described in detail below. Optionally, the position limiting structure 43 includes at least one position limiting rod 430 provided at the terminal end of the corresponding outer connection unit 470. Referring to FIG. 9a, in this embodiment, the position limiting rod 430 is substantially T-shaped, and includes a rod portion 431 for connecting with the corresponding outer connection unit 470, and an engaging portion 432 enlarged from a terminal end of the rod portion 431. In this embodiment, the engaging portion 432 of the position limiting rod 430 has a substantially circular axial cross section. It is to be understood that, in other embodiments, the engaging portion can have other shapes. For example, as shown in FIG. 9b, in some embodiments, the axial cross section of the engagement portion 432B is rectangular. In some other embodiments, as shown in FIG. 9c, the axial cross section of the engaging portion 432C is semi-circular.

Referring again to FIGS. 3, 5 and 7, in this embodiment, the outer skirt section 42 of the outer frame 40 extends from a substantially middle portion of the outer body section 41 of the outer frame 40 outwardly away from the central axis L of the outer frame 40 and towards the inner skirt section 32. FIG. 7 shows the outer skirt section 42 extending substantially obliquely upwards and outwards. Preferably, the outer skirt section 42 extends outwardly from where the top section 46 and the bottom section 45 meet. In other words, the outer skirt section 42 extends outwardly from the portion of the outer body section 41 having the maximum diameter, and the support section of the outer body section 41 between the outer skirt section 42 and the inner skirt section 32 for forming the receiving space 50 is the top section 46.

It will be appreciated that, in other embodiments, the outer skirt section 42 can extend outwardly from other portion of the outer body section 41, such as from a portion of the top section 46 adjacent to the outflow end of the top section 46. In this case, a part of the top section 46 is configured as the support section for forming the receiving space.

Preferably, the minimum axial spacing H between the inner skirt section 32 and the outer skirt section 42 is in the range of 5 mm to 15 mm, which is approximately equal to the thickness of the native valve annulus MVA, so that the valve annulus MVA can be stably received in the receiving space 50, thereby preventing the movement of the stent of prosthetic heart valve 10 due to an excessive gap and improving the positioning effect.

Preferably, the angle A6 between the starting end 421 of the outer skirt section 42 (i.e., the end close to the outer body section 41, which is also the connection end) or the tangent line of the starting end 421 of the outer skirt section 42 and the central axis L of the outer frame 40 ranges from 60 degrees to 120 degrees. The angle A7 between the terminal end 422 of the outer skirt section 42 (i.e. the end away from the outer body section 41, which is also the free end) or the tangent line of the terminal end 422 of the outer skirt section 42 and the central axis L of the outer frame 40 ranges from 60 degrees to 120 degrees.

More preferably, the outer skirt section 42 extends outward in an arc shape from the outer body section 41, and the included angle A8 between the tangent line of the substantially middle portion of the outer skirt section 42 and the central axis L of the outer frame 40 ranges from 30 degrees to 90 degrees, which allows the outer skirt section 42 to extend radially and outwardly with an arc transition between the starting end 421 and the terminal end 422.

More preferably, the maximum outer diameter D3 of the outer skirt section 42 (i.e., the diameter of the terminal end 422 of the outer skirt section 42 in this embodiment) ranges from 40 mm to 70 mm, which is about 10 mm greater than the inner diameter of a normal native valve annulus MVA, thereby effectively preventing the movement of the prosthetic heart valve 100 towards the atrial side.

Referring to FIGS. 7 and 10a, in this embodiment, the outer skirt section 42 includes a plurality of support units 423 that are uniformly spaced from each other in the circumferential direction. Each support unit 423 includes two support rods 424. Each support rod 424 includes a first end 425 away from the outer body section 41 and connected with another support rod 424, and a second end 426 connected with the outer body section 41. In this embodiment, the second end 426 of each support rod 424 is connected with a substantially middle portion of a corresponding strut 412 of the middle wave bar 443B. The first ends 425 of the two support rods 424 of each support unit 423 are connected at a joint. Preferably, the joint has a blunt end, for example, the joint has an arc shape, to reduce injury and damage to the native valve annulus MVA. The second ends 426 of adjacent two support rods 424 of adjacent two support units 423 are spaced apart. Preferably, two adjacent support rods 424 of two adjacent support units 423 are spaced apart by one cell 441. It will be appreciated that in other embodiments, the outer skirt section 42 can have other configurations as long as it can support the ventricular side of the valve annulus MVA.

For example, as shown in FIG. 10b, in other embodiments, the two support rods 424B of the support unit 423B can respectively extend from the first ends 425B thereof in an arc shape away from the axis of symmetry of the support unit 423B (i.e., the axis of symmetry of the two support rods 424B), which can increase the support area of the support unit 423B to the native valve annulus MVA. As shown in FIG. 10c, in other embodiments, each support rod 424C of the support unit 423C can have a curved extension from its first end 425C away from the axis of symmetry of the support unit 423C. As shown in FIG. 10d, in other embodiments, each support rod 424D of the support unit 423D can extend linearly from its first end 425D away from the axis of symmetry of the support unit 423D.

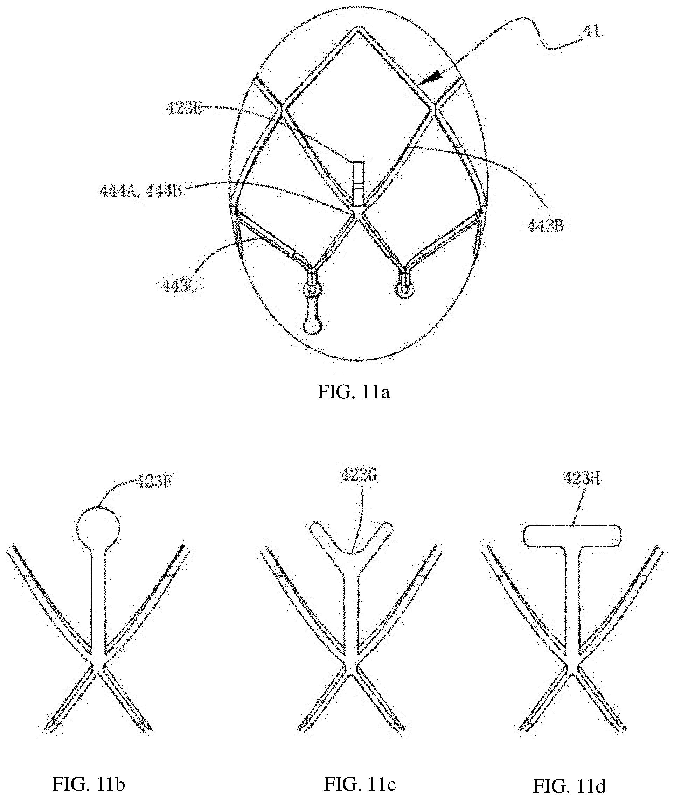

For example, as shown in FIG. 11a, in other embodiments, each support unit 423E of the outer skirt section no longer includes two support rods 424. Instead, each support unit 423E is substantially rod-shaped. Preferably, the end of each rod-shaped support unit 423E is formed as a blunt end extending arcuately outward with respect to the outer body section 41. Preferably, each support unit 423 extends outward from a corresponding valley 444B of the middle wave bar 443B or a corresponding peak 444A of the lower wave bar 443C.

For example, as shown in FIG. 11b, in other embodiments, the terminal end of each rod-shaped support unit 423F can be formed as a rounded blunt end. As shown in FIG. 11c, in other embodiments, the terminal end of each rod-shaped support element 423G can be formed as a substantially "C" shaped blunt end. As shown in FIG. 11d, in other embodiments, the end of each rod-shaped support unit 423H can be formed as a rectangular blunt end.

Optionally, the outer skirt section 42, and the outer body section 41 are formed separately, and the outer skirt section 42 is configured to be connected to the outer body section 41, for example by crimping, riveting, welding or sewing.

Preferably, the outer skirt section 42 and the outer body section 41 are formed in one piece. That is, the outer frame 40 is preferably a single piece.

Optionally, the outer frame 40 or sections of the outer frame 40 can be formed by laser cutting a tube(s) having shape memory property such as a nickel-titanium tube (s) and heat setting the same into the desired shape. Alternatively, the outer frame 40 or sections of the outer frame 40 may be formed by braiding a wire(s) having shape memory property such as a nickel-titanium alloy wire(s) and heat setting the same into the desired shape.

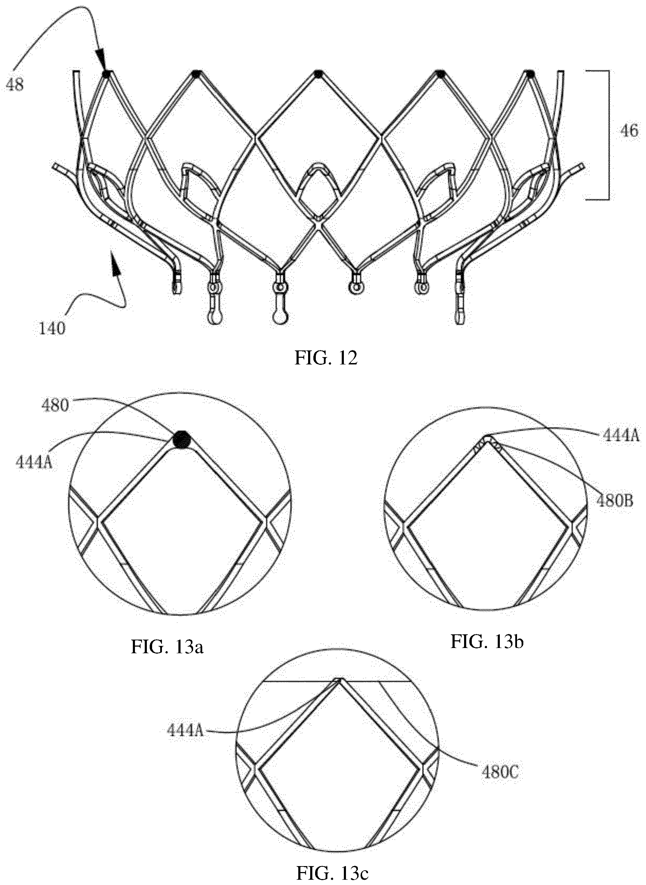

Referring to FIG. 12, the prosthetic heart valve according to the second embodiment of the present disclosure is similar to the prosthetic heart valve 100 according to the first embodiment, and the same aspects will not be repeated here. The main difference between the prosthetic heart valve according to the second embodiment of the present disclosure and the prosthetic heart valve 100 according to the first embodiment of the present disclosure is that: the outer frame 140 of the prosthetic heart valve according to the second embodiment of the present disclosure is further provided with a marking mechanism 48, in order to determine that actual position of the prosthetic heart valve when the prosthetic heart valve is implanted in the human body, in particular by minimally invasive interventional procedure. The material of the marking mechanism 48 can be a radiopaque material visible under X-rays, such as tungsten, gold, platinum, or tantalum.

Referring to FIGS. 12 and 13a, the marking mechanism 48 of the prosthetic heart valve according to the present embodiment includes a plurality of marking points 480. The marking points 480 can be fixed to the inflow end of the outer frame 140 by, for example, crimping. Preferably, the plurality of marking points 480 are evenly spaced from each other and provided at the plurality of peaks 444A at the inflow end of the top section 46 of the outer frame 140. It will be appreciated that in other embodiments, the plurality of marking points can be provided at other portions of the stent of prosthetic heart valve. For example, the plurality of marking points 480 can be provided on the inner skirt section 32 of the inner frame 30. Alternatively, only one marking point 480 can be provided.

In other embodiment, the marking mechanism can have other configurations. For example, as shown in FIG. 13b, in other embodiment, the marking mechanism can include a plurality of marking segments 480b. Each marking segment 480B can be formed by a length of marking wire / line fixed to the respective peak 444A at the inflow end of the top section 46 by, for example, winding. For example, as shown in FIG. 13c, in other embodiments, the marking mechanism can be configured as a continuous, complete marking ring 480C. The marking ring 480C can be formed by a length of marking wire / line passing through the plurality of peaks 444A at the inflow end of the top section 46 by, for example, sewing or the like and being fixed to the inflow end of the top section 46. It will be appreciated that in other embodiments, the marking mechanism can be formed as a continuous, but incomplete, such as an arcuate, or semi-circular configuration.

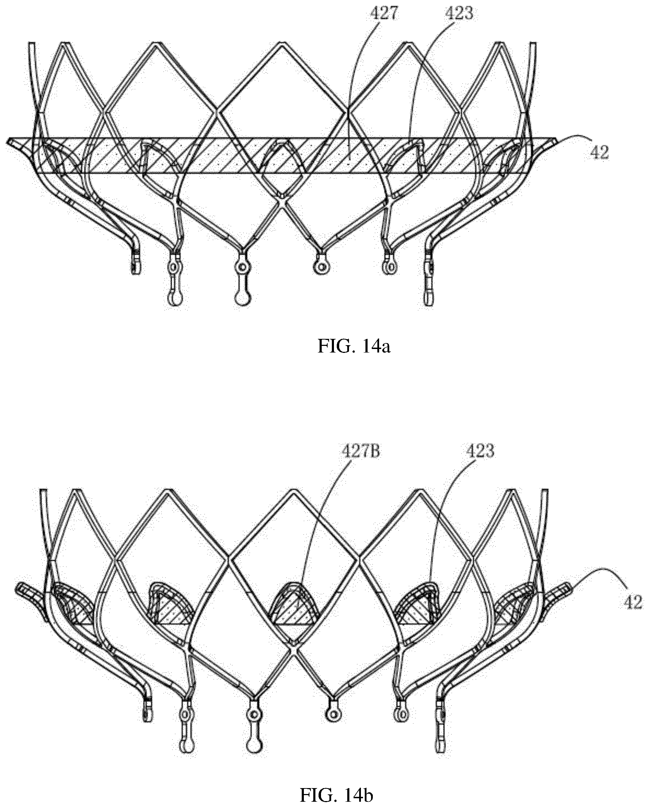

Referring to FIG. 14a, the prosthetic heart valve according to the third embodiment of the present disclosure is similar to the prosthetic heart valve 100 according to the first embodiment, and the same aspects will not be repeated here. The main difference between the prosthetic heart valve according to the third embodiment of the present disclosure and the prosthetic heart valve 100 according to the first embodiment is that: the outer skirt section 42 of the prosthetic heart valve according to the third embodiment of the present disclosure is covered with a protective film 427, to further reduce injury and damage to the valve annulus MVA from the outer skirt section 42. The protective film 427 is preferably made of PET, PTFE, or the like. As can be seen from FIG. 14a, the protective film 427 in this embodiment is formed as a continuous ring. In other words, the protective film 427 in the present embodiment covers not only all of the support units 423 but also gaps between adjacent support units 423.

It will be appreciated that in other embodiments, the protective film can take other configurations. For example, as shown in FIG. 14b, in other embodiments, the protective film 427B can include a plurality of segments that are circumferentially spaced from each other, with each segment covering a corresponding support unit 423, so that injury to the valve annulus MVA from the outer skirt section 42 can also be prevented.

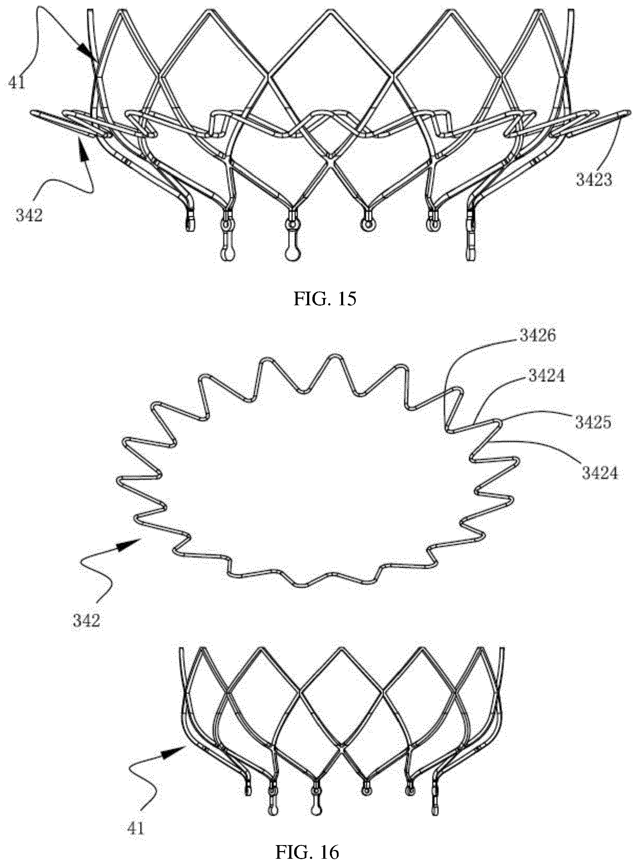

Referring to FIG. 15 and FIG. 16, the prosthetic heart valve according to the fourth embodiment of the present disclosure is similar to the prosthetic heart valve 100 according to the first embodiment, and the same aspects will not be repeated here. The main difference between the prosthetic heart valve according to the fourth embodiment of the present disclosure and the prosthetic heart valve 100 of the first embodiment is that: the outer skirt section 342 of the prosthetic heart valve according to the fourth embodiment of the present disclosure is configured as a continuous ring. In this embodiment, the first ends 3425 of the two support rods 3424 of each support unit 3423 are connected to each other, and the second ends 3426 of the two adjacent support rods 3424 of two adjacent support units 3423 are also connected to each other. In particular, the first ends 3425 of the two support rods 3424 of each support unit 3423 respectively extend outwardly to the radially outer side of the outer body section 41 and are connected, preferably to form, for example, an arc blunt end, to reduce the injury to the native valve annulus MVA. The second ends 3426 of the two support rods 3424 of each support unit 3423 respectively extend inwardly to the radially inner side of the outer body section 41 and are connected to the second ends 3426 of the adjacent support rods 3424 of the adjacent support units 3423, and also preferred to form, for example, arc blunt ends.

Preferably, the outer skirt section 342 in this embodiment is formed separately from the outer body section 41, and the outer skirt section 342 is configured to be connected to the outer body section 41, for example, by sewing. More preferably, the outer skirt section 342 is formed by braiding a nickel-titanium alloy wire(s) and heat setting the same into the desired shape. The outer body section 41 is formed by laser cutting a nickel-titanium tube (s) and heat setting the same into the desired shape.

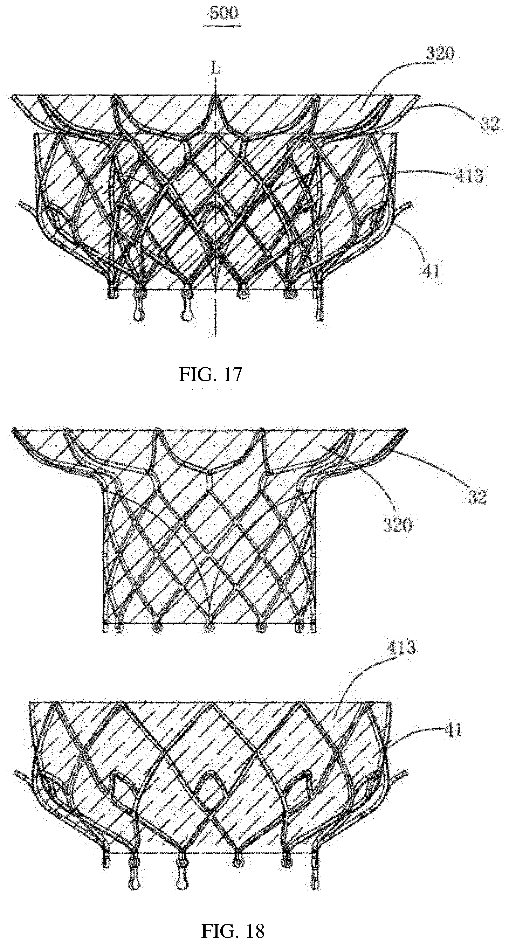

Referring to FIGS. 17 to 19, the prosthetic heart valve 500 according to the fifth embodiment of the present disclosure is similar to the prosthetic heart valve 100 according to the first embodiment, and the same aspects will not be repeated here. The main difference between the prosthetic heart valve 500 according to the fifth embodiment of the present disclosure and the prosthetic heart valve 100 according to the first embodiment of the present disclosure is that: the outer body section 41 of the outer frame of the prosthetic heart valve 500 according to the fifth embodiment of the present disclosure is also covered with a flow blocking film 413. The material of the flow blocking film 413 is preferably PET, PTFE, or the like. A double sealing, including the sealing to the atrial side of the valve annulus MVA by the flow blocking film 320 of the inner skirt section 32, and the sealing to the ventricular side of the valve annulus MVA by the flow blocking film 413 of the outer body section 41, is provided, thereby further enhancing the sealing effect of the prosthetic heart valve 500 and reducing the risk of perivalvular leakage.

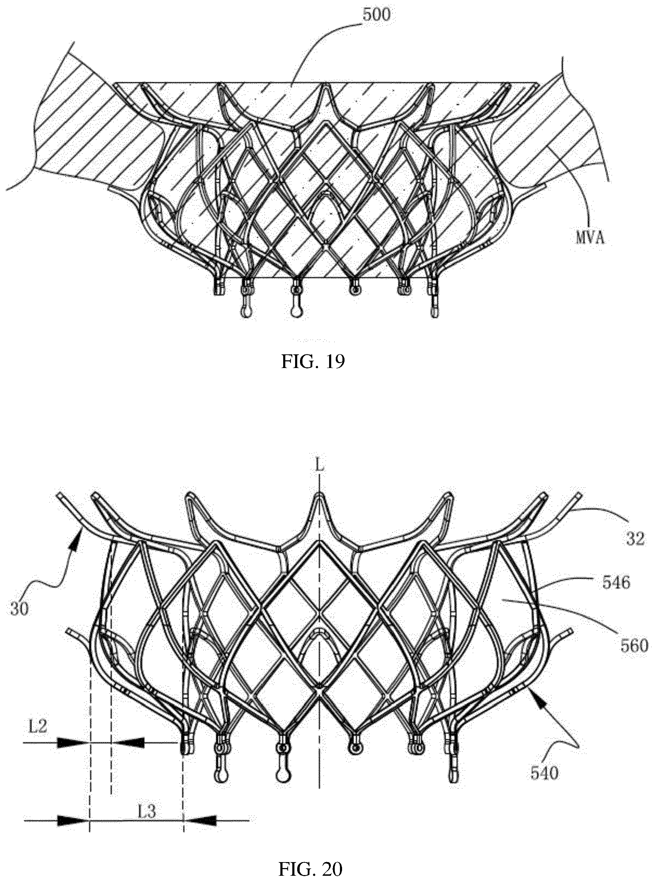

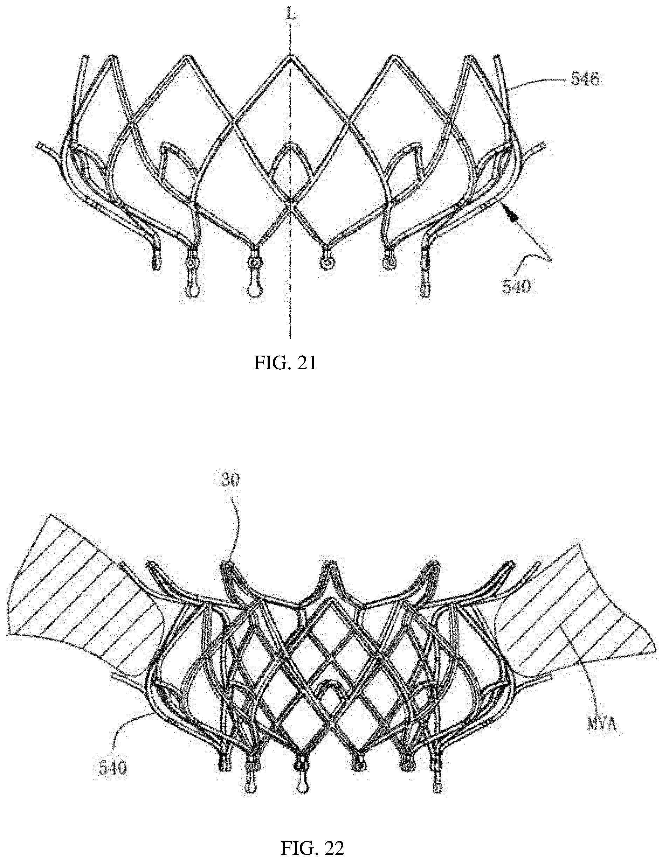

Referring to FIGS. 20 to 22, the prosthetic heart valve according to the sixth embodiment of the present disclosure is similar to the prosthetic heart valve 100 according to the first embodiment, and the same aspects will not be repeated here. The main difference between the prosthetic heart valve according to the sixth embodiment of the present disclosure and the prosthetic heart valve 100 according to the first embodiment of the present disclosure is that: the diameters of different portions of the top section 546 of the outer frame 540 of the prosthetic heart valve according to the sixth embodiment of the present disclosure are different. The top section 546 of the outer frame 540 in this embodiment extends not only axially towards the inner skirt section 32 but also inwardly toward the central axis L of the outer frame 540. In other words, in this embodiment, the diameter of the top section 546 of the outer frame 540 gradually decreases from the outflow end thereof to the inflow end thereof, so that the top section 546 has an angled configuration converging toward the central axis L of the outer frame 540, which further improves the compliance of the outer frame 540 to the valve annulus MVA so that when the prosthetic heart valve is implanted, the top section 546 of the outer frame 540 can receive the valve annulus MVA without or with only small deformation.

Preferably, the radial distance L2 between the outflow end and the inflow end of the top section 546 is smaller than the radial distance L3 between the outflow end of the top section 546 and the inner body section 31, so that a radial gap 560 is formed between the top section 546 and the inner body section 31 to prevent deformation of the inner frame 30 of the implanted prosthetic heart valve 100 under the pressure of the outer frame 540, thereby further reducing the risk of perivalvular leakage.

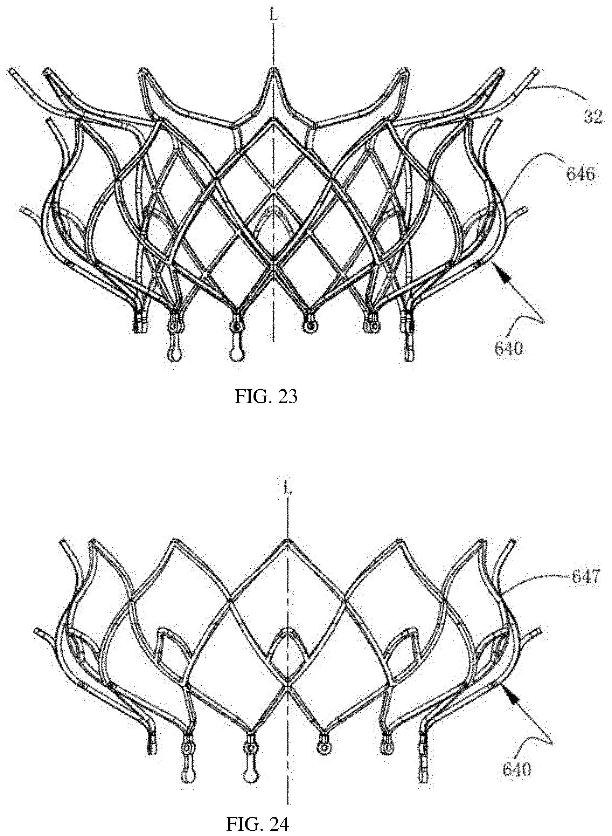

Referring to FIGS. 23 to 25, the prosthetic heart valve according to the seventh embodiment of the present disclosure is similar to the prosthetic heart valve 100 according to the first embodiment, and the same aspects will not be repeated here. The main difference between the prosthetic heart valve according to the seventh embodiment and the prosthetic heart valve 100 according to the first embodiment is that: the top section 646 of the outer frame 640 of the prosthetic heart valve according to the seventh embodiment no longer extends with the same diameter. The top section 646 of the outer frame 640 in this embodiment extends axially towards the inner skirt section 32 first inwardly towards the central axis L of the outer frame 640 and then outwardly away from the central axis L of the outer frame 640 so as to form a recess 647 in a round. The axial cross section of the top section 646 in this embodiment is substantially S-shaped. The recess 647 allows the valve annulus MVA to conform to the top section 646 better, improving the compliance of the top section 646 to the valve annulus MVA and thus improving the positioning stability of the prosthetic heart valve.

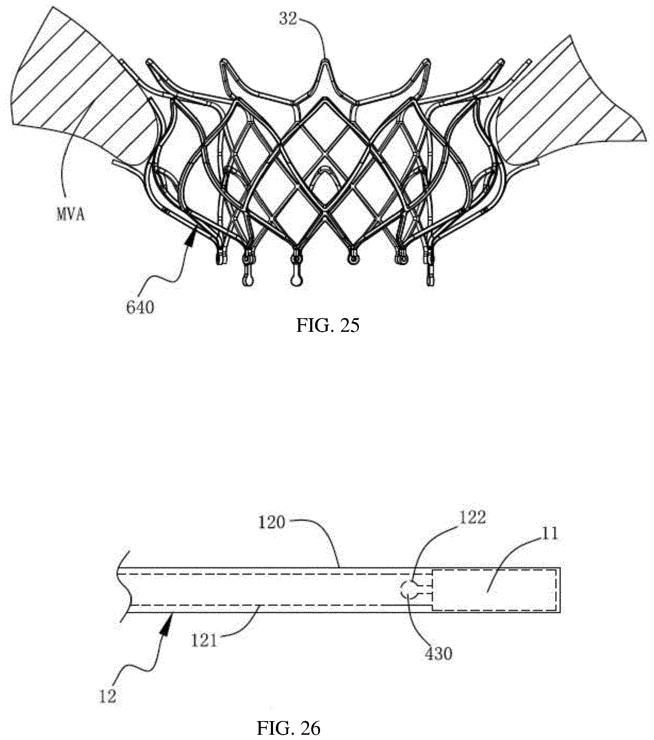

Referring to FIG. 26, a prosthetic heart valve replacement system according to an embodiment of the present disclosure includes a prosthetic heart valve 11 and a delivery device 12 for delivering the prosthetic heart valve 11. The prosthetic heart valve 11 can be any of the prosthetic heart valves described above. The prosthetic heart valve 11 has a delivery condition radially compressed as shown in FIG. 26 and a natural condition radially expanded as shown in the figures showing the prosthetic heart valves described above.

The delivery device 12 includes an outer sheath 120 and an inner core 121 extending through the outer sheath 120. The inner core 121 and the outer sheath 120 are axially movable relative to each other. The prosthetic heart valve 11 in the delivery condition is radially compressed and received in a gap between the distal portion of the inner core 121 and the distal portion of the outer sheath 120. Preferably, the distal portion of the inner core 121 is provided with a position limiting groove 122 for receiving the position limiting rod 430 of the prosthetic heart valve 11. The position limiting groove 122 has a shape complementary to the shape of the position limiting rod 430 describe above.

In procedure, when the operator pulls the outer sheath 120 proximally or pushes the inner core 121 distally to preliminarily release the prosthetic heart valve 11, the position limiting rod 430 of the prosthetic heart valve 11 is still tightly engaged with the position limiting groove 122 of the inner core 121 under the constraint of the outer sheath 120, so that the prosthetic heart valve 11 can be effectively prevented from falling off from the inner core 121, and thus the operator can observe and adjust the position of the prosthetic heart valve 11 through medical imaging. After the prosthetic heart valve 11 is adjusted to the desired position for release, the outer sheath 120 is further pulled proximally or the inner core 121 is further pushed distally so that the outer sheath 120 no longer constrains the position limiting rod 430, the position limiting rod 430 is then released from the position limiting groove 122 due to the radial expansion of the stent of prosthetic heart valve itself, thereby completely releasing the prosthetic heart valve 11.

Finally, it should be noted that the above embodiments are only used to illustrate the technical solutions of the present disclosure, and are not limited thereto. Although the present disclosure has been described in detail with reference to the foregoing embodiments, it should be understood by those skilled in the art that the technical solutions described in the foregoing embodiments can still be modified, or some or all of the technical features thereof can be substituted, and these modifications or substitutions fall in the protection scope of the present disclosure.

## Claims

1. A stent of prosthetic heart valve, comprising an inner frame and an outer frame connected with each other; the outer frame comprises an outer body section, and an outer skirt section extending from the outer body section and located at a radially outer side of the outer body section, wherein the outer skirt section is located between an inflow end and an outflow end of the outer body section; the inner frame comprises an inner body section at least partially within the outer frame, and an inner skirt section projecting radially outside the inner body section from an inflow end of the inner body section, wherein the inner skirt section projects radially outside the inflow end of the outer body section, so that the outer skirt section, the inner skirt section, and a section of the outer body section between the outer skirt section and the inner skirt section together form a receiving space which opens radially outward.

2. The stent of prosthetic heart valve according to claim 1, wherein the inflow end of the outer body section is free and suspended, and a radial gap is formed between the section of the outer body section between the outer skirt section and the inner skirt section and the inner body section, and the inner skirt section is covered with a flow blocking film.

3. The stent of prosthetic heart valve according to claim 2, wherein the outer body section comprises a bottom section extending outwardly away from a central axis of the outer frame and towards the inner skirt section simultaneously, and a top section extending from an inflow end of the bottom section further toward the inner skirt section; wherein the section of the outer body section between the outer skirt section and the inner skirt section is at least partially formed by the top section.

4. The stent of prosthetic heart valve according to claim 3, wherein an outflow end of the bottom section is fixedly connected with an outflow end of the inner body section.

5. The stent of prosthetic heart valve according to claim 3, wherein the top section extends axially toward the inner skirt section and inwardly toward the central axis of the outer frame simultaneously; or the top section extends axially toward the inner skirt section, while first extending inwardly toward the central axis of the outer frame and then outwardly away from the central axis of the outer frame so as to form a recess in a round.

6. The stent of prosthetic heart valve according to claim 3, wherein the top section has an O-shaped or D-shaped outer peripheral profile.

7. The stent of prosthetic heart valve according to claim 3, wherein an included angle formed by two tangent lines of an outflow end of the bottom section that are radially opposite to each other ranges from 90 degrees to 150 degrees.

8. The stent of prosthetic heart valve according to claim 4, wherein the inner body section has a higher deformation resistance than the outer body section, and the bottom section of the outer body section has a higher deformation resistance than the top section of the outer body section.

9. The stent of prosthetic heart valve according to claim 8, wherein the inner body section having a higher deformation resistance than the outer body section is achieved by at least one of a group consisting of: area of openings of cells of the inner body section being smaller than that of the outer body section, rigidity of material of the inner body section being greater than that of the outer body section, size of struts of the inner body section being greater than that of the outer body section; the bottom section of the outer body section having a higher deformation resistance than the top section of the outer body section is achieved by at least one of a group consisting of: area of openings of cells of the bottom section being smaller than that of the top section, rigidity of material of the bottom section being greater than that of the top section, size of struts of the bottom section being greater than that of the top section.

10. The stent of prosthetic heart valve according to claim 1, wherein the minimum axial distance between the inner skirt section and the outer skirt section ranges from 5 mm to 15 mm.

11. The stent of prosthetic heart valve according to claim 1, wherein the outer skirt section extends outward from a portion of the outer body section having the largest diameter.

12. The stent of prosthetic heart valve according to claim 1, wherein an angle between a starting end of the outer skirt section or a tangent line to the starting end of the outer skirt section and a central axis of the outer frame ranges from 60 degrees to 120 degrees; an angle between a terminal end of the outer skirt section or a tangent line to the terminal end of the outer skirt section and the central axis of the outer frame ranges from 60 to 120 degrees.

13. The stent of prosthetic heart valve according to claim 1, wherein an angle between a starting end of the inner skirt section or a tangent line to the starting end of the inner skirt section and a central axis of the inner frame ranges from 45 degrees to 90 degrees.

14. The stent of prosthetic heart valve according to claim 2, wherein the inner body section is covered with a flow blocking film, and the outer body section is also covered with a flow blocking film.

15. The stent of prosthetic heart valve according to claim 1, further comprising a marking mechanism disposed on the outer frame and / or the inner frame.

16. The stent of prosthetic heart valve according to any one of claims 1 to 15, wherein the outer skirt section comprises a plurality of support units distributed in a circumferential direction, each of said support units comprises two outwardly extending support rods, each of said support rods comprises a first end away from said outer body section and a second end adjacent to and connected to said outer body section, the first ends of the two support rods of each support unit are connected and smoothly transited to each other, and the second ends of two adjacent support rods of two adjacent support units are spaced apart from or connected with each other.

17. The stent of prosthetic heart valve according to any one of claims 1 to 15, wherein the outer skirt section comprises a plurality of support units spaced from each other in a circumferential direction, each of the support units is substantially rod-shaped, and a free end of the support unit is a blunt end.

18. The stent of prosthetic heart valve according to any one of claims 1 to 15, wherein the outer skirt section is covered with a protective film.

19. A prosthetic heart valve, comprising at least two prosthetic leaflets and the stent of prosthetic heart valve according to any one of claims 1 to 18, wherein the prosthetic leaflets are fixedly connected within the inner body section of the inner frame of the stent of prosthetic heart valve, and edges of the at least two prosthetic leaflets are circumferentially engaged with each other.

20. A prosthetic heart valve replacement system, comprising the prosthetic heart valve according to claim 19 and a delivery device for delivering the prosthetic heart valve; the prosthetic heart valve has a delivery condition being radially compressed and a natural condition being radially expanded; the delivery device comprises an outer sheath and an inner core passing through the outer sheath, and the inner core and the outer sheath are axially movable relative to each other; the prosthetic heart valve is radially compressed and received in a gap between a distal portion of the inner core and a distal portion of the outer sheath.
